# EUROPEAN PATENT SPECIFICATION

(11) **EP 2 734 844 B1**
(45) Date of publication and mention of the grant of the patent: **12.09.2018**
(21) Application number: 12814540.6
(22) Date of filing: 12.07.2012
(51) Int. Cl.: G01N 33/543

(54) **METHOD FOR THE DETECTION OF AN ANALYTE IN A SAMPLE**
VERFAHREN ZUM NACHWEIS EINES ANALYTEN IN EINER PROBE
PROCÉDÉ DE DÉTECTION D'UNE SUBSTANCE À ANALYSER DANS UN ÉCHANTILLON

(30) Priority: 20.07.2011 SE 1100553
(43) Date of publication of application: 28.05.2014
(73) Proprietor: Glycorex Transplantation AB, 223 70 Lund (SE)
(72) Inventor: NILSSON, Kurt, S-226 53 Lund (SE); KRONBLAD, Åsa, S-238 43 Oxie (SE)
(74) Representative: AWA Sweden AB
(86) International application number: PCT/SE2012/050830
(87) International publication number: WO 2013/012387

(56) References cited:
- EP-A1- 0 241 140
- EP-A1- 1 619 500
- WO-A1-89/00446
- WO-A1-2011/046504
- WO-A2-03/090605
- WO-A2-2006/012486
- DE-A1- 10 325 919
- US-A- 5 164 298
- US-A1- 2002 160 363
- US-B1- 6 444 655
- CLAVIJO A ET AL.: 'Simultaneous detection of antibodies to foot-and-mouth disease non-structural proteins 3ABC, 3D, 3A and 3B by a multiplexed Luminex assay to differentiate infected from vaccinated cattle' VACCINE vol. 24, no. 10, 2006, pages 1693 - 1704, XP028010520
- MOORTHY J ET AL.: 'Microfluidic tectonic platform:A colorometric, disposable botulinium toxin enzyme-linked immunosorbent assay system' ELECTROPHORESIS vol. 25, 2004, pages 1705 - 1713, XP055142724
- MATHIALAGAN S ET AL.: 'Expression, purification and functional characterization of IkappaBkinase-2 (IKK-2) mutants' PROTEIN EXPRESSION AND PURIFICATION vol. 72, 2010, pages 254 - 261, XP027052639
- WONG J ET AL.: 'Rapid detection of antibodies in sera using multiplexed self-assembling bead arrays' J IMMUNOLOGY METHODS vol. 350, 2009, pages 171 - 182, XP026684074
- OKU ET AL.: 'Development of a Highly Sensitive Bead-ELISA to Detect Bacterial Protein Toxins' MICROBIAL IMMUNOL vol. 32, 1988, pages 807 - 816, XP001094595

## Description

### Technical field of the Invention

The present invention refers to an improved method for the detection of at least one analyte in a sample.

### Background Art

It is well-known to purify antibodies and/or antigens from a sample in a conventional analysis plate, e.g. an ELISA plate, containing microparticles or beads, with ligands bound thereto located in the bottom of the wells on the analysis plate. Antibodies and/or antigens present in the sample added are specifically bound to the ligands present on the microparticles or on the beads. Thereafter, non-bound sample components still present in the wells are washed out from the wells by the addition of a wash liquid. Thereafter, the antibodies/proteins or antigens are eluated from the ligands by the addition of a buffer, and separated purified antibodies, proteins and/or antigens are collected and analyzed.

It is also known to detect specific analytes, e.g. proteins, in a sample by use of an ELISA plate having a porous filter in the bottom of each well and a layer of microparticle or porous beads located above said porous filter. The microparticles or porous beads contain a covalently bound ligand, which is able to bind to the analyte. When a sample is added to the well of the ELISA plate, the analyte is specifically bound to the ligand. Non-bound analyte components still present in the well are washed out from the plate through the porous filter by the addition of a wash liquid. For detection and quantification of the bound analyte, different techniques are used. The microparticles or beads can be removed and analyzed by for example flow cytometry. Alternatively, the analyte can be eluted from the microparticles by adding a buffer for elution and eluting the analyte through the filter and thereafter determining the quantity of the analyte by for example sodium dodecyl sulfate polyacrylamide gel electrophoresis, or by traditional ELISA techniques. If the analyte is radiolabelled or reacted with a radiolabelled substance, the analyte is determined using scintillation counting. As another alternative, a scintillation liquid is added to the wells and the scintillation is measured.

Several different pathogenic bacteria and virus bind to specific carbohydrate receptors in humans where the carbohydrate acts like an anchor point for the bacteria, virus or bacterial toxin. EHEC, enterohemorrhagic Escherichia coli, is one of several examples of micro-organisms which either itself or via toxins binds to carbohydrate receptors in human beings. EHEC produces a toxin called Shiga toxin, which also may be produced by several other types of infectious bacteria, e.g. Shigella. The Shiga toxin binds to Gb3 (globotriaosylceramide) structures, in which the disaccharide unit in Gb3, i.e. Galα1-4Gal, is regarded to be smallest saccharide structure to which the Shiga toxin binds. Gal means D-galactose, and α1-4 means the glycosidic bond between the two galactose units. The Galα1-4Gal structure is also of importance for example in urinary tract infections caused by the binding of Galα1-4Gal specific E.coli. Several other carbohydrate structures such as structures containing one or several of for example Man (D-mannose), Fuc (L-fucose), sialic acid (for example N-acetylneuraminic acid), Glc (D-glucose), GlcNAc (N-acetyl-D-glucosamine) and/or GalNAc (D-N-acetyl-galactosamine) for example, have been implicated in bacterial and viral infections.

Carbohydrate structures of importance in connection with various forms of human cancer (for example Galili-antigens) have been identified, as well as antibodies specific for such structures. Also blood group structures have been implicated in bacterial and viral infections. Human antibodies specific for blood group A and B structures are important for example in blood group incompatible translplantation, in blood group incompatible blood transfusions and in immunoglobulin preparations for injection (IVIG).

With a view to treating viral and bacterial infections, such as HUS, hemolytic uremic syndrome, antibiotic treatments, in some cases together with repeated blood transfusions and in some cases together with repeated plasma exchanges, are applied. However, these measures are not specifically acting on the toxin and may lead to side-effects and/or a non-desired treatment effect. Instead, a more specific treatment is desired. Further, it is of interest with an access to better detection methods, e.g. for screening of the toxin(s) and bacteria involved with a view to diagnosing and properly treating the disease condition in question.

Antibodies are of interest in a range of conditions. Several autoimmune diseases involve autoantibodies, for example anti-GM1 antibodies. In blood group incompatible transplantation, for example, it is important to determine the levels of recipient antibodies specific for the donor blood group antigens, pre- and posttransplant. The present methods for determination blood group specific antibodies rely on red blood cells as obtained from human blood, or modified such cells, and using for example gel cards or expensive flow cytometry for their determination. For the HLA sensitized transplant recipient, the levels of different HLA antibodies are important.

The above examples of analytes of interest are mentioned as examples and are not intended to limit the scope of the invention.

The known detection and purification methods based on various types of beads or suspensions have different problems. Various methods for detection have been developed, but often involve expensive equipment, and/or are time consuming, or involve radioactive labels for detection. Traditional ELISA with ligands/primary antibodies non-covalently or covalently bound to the plastic surfaces of the wells in the ELISA plates, requires relatively long incubation times for the binding reactions. Also, the surfaces used for binding of the ligand/primary antibody often introduce non-specific adsorption effects, especially for smaller ligands. For larger ligands such as proteins there is a risk that the structure of the protein is more or less impaired. Also, the surface of the wells is limited, resulting in limited sensitivity, a problem when the analyte is present in a relatively low concentration in the sample.

Thus, there is a need of an improved method for the detection of analytes in a sample when using an analysis plate technique.

EP 0 241 140 A1 refers to a method of detecting the presence of a cell, virus or circulating body component or an antibody thereto in a sample.

WO 89/00446 discloses solid supports including an immobilized flocculating agent which can be used as a separation and assay media in the field of affinity separations.

WO 2006/012486 discloses an improved system for efficiently and accurately performing immunoassays, such as ELISAs, and comprises an immunoassay assembly including a flow-through unit and an aspiration pump.

### Summary of the Invention

The object of the present invention is to reduce the above-mentioned problems associated with known products and methods used for detection and quantification. This object is achieved by the method according to the present invention.

In one embodiment the present invention relates to a method for the detection of at least one analyte in a sample, wherein it comprises the steps of:
a) providing a layer comprising a porous matrix having the form of several porous agarose gel beads and having ligands bound to the porous matrix both on the surface of the matrix and inside the pores of the matrix above a filter constituting the bottom of several wells of an ELISA plate, wherein the pores of said porous agarose gel beads allow entrance of proteins or antibodies up to at least one million Daltons into said pores, wherein said filter does not allow passage of said porous matrix having a ligand bound thereto and wherein a part of the ELISA plate below the filter has a funnel shape narrowing downwards to a smaller opening,
b) adding a sample containing said at least one analyte to be detected, said at least one analyte having the ability to specifically bind to said at least one ligand,
c) adding a wash solution with a view to washing out non-bound sample components from the ELISA plate through the filter,
d) adding enzyme-linked antibodies or antigens having the ability to specifically bind to said at least one analyte,
e) adding a wash solution with a view to washing out non-bound enzyme-linked antibodies or antigens from the analysis plate through the filter,
f) adding a substrate specific for the enzyme, wherein a product is formed by a reaction between the enzyme and the substrate, wherein the product formed is either subjected to direct spectrophotometic measurement of the adsorption of the wells in the ELISA plate or is washed from the well with a wash solution through the filter in the bottom of the well and is collected as an eluate below the ELISA plate,
g) determining the quantity of said at least one analyte by measuring a signal related to the product.

The measurement of the product produced by the enzyme is proportional to the amount of the analyte bound to the beads in each well. The measurement of the product can for example be made with a conventional ELISA plate reader, either by direct spectrophotometric measurement of the absorption of the wells in the plate, wherein steps a)-f) above are performed, or by allowing the product formed in the enzyme reaction f) above to pass through the filter in each well of the ELISA plate down to corresponding wells of a second ELISA plate and measurement of the absorbance of the wells of this second plate.

A product kit is also disclosed, said kit comprising one or more of the following components:
1) a layer comprising a porous matrix having at least one ligand bound thereto, situated above a porous filter constituting the bottom of at least one well of an assay plate, wherein said filter does not allow passage of said porous matrix having a ligand bound thereto,
2) the ligand in a) above having the ability to specifically bind at least one analyte in the sample,
3) at least one enzyme-linked antibody, or enzyme-linked antigen, having the ability to specifically bind to said at least one analyte,
4) at least one wash solution with a view to washing out non-bound enzyme-linked antibodies, or enzyme-linked antigens, from the analysis plate through the filter,
5) at least one substrate specific for the enzyme which is converted to at least one product by the enzyme, the product being able to be detected spectrophotometrically or by other method,
6) optionally a second plate, having the same number of wells as the first plate, but not having a filter in the bottom of the wells. The wells of this second plate can be placed below the corresonding wells of the plate in 1) above, allowing the product in 5) above to pass down through the filter of the first plate down to the corresponding wells of the second plate. The absorption of the product, now in the corresponding wells of the second plate, is read spectrophotometrically in an ELISA plate reader.

### Detailed Description of Preferred Embodiments

The present invention relates to an improved method for detection of a least one analyte in a sample, e.g. antibodies, proteins, peptides, and bacterial toxins as exemplified above.

A porous matrix having a ligand bound thereto is used.

Examples of a preferred matrix is an agarose based matrix, e.g. cross-linked agarose which normally is in the form of beaded gel particles. The agarose matrix is known to be porous or macroporous, thus containing pores which allow entrance of proteins or antibodies up to at least one million Daltons into said pores, where a large part of ligand is bound. Further non-limiting examples of agarose are commercially available, for example so called CL-Sepharose 2B, 4B or 6B, or similar so called Fast Flow variants and other similar variants. The matrix can be chosen by the expert in the field.

There are other matrixes based on e.g. cellulose, cross-linked cellulose, and plastic porous polymers and porous filter materials used in e.g. hemofiltration and plasma exchange.

The linkage between the ligand and the matrix is preferentially covalently stable, and examples thereof are an amide, an N-C or an O-C linkage.

The former linkage is preferentially formed by reacting an activated carboxyl group (activated with a carbodiimide and/or an N-hydroxysuccinimide) on a ligand, or a matrix, with an amino group on a matrix, or a ligand. The pH, solvent, temperature, concentrations of reagents, matrix and ligand, reaction temperature, and washing procedures, so called coupling procedures, are selected by the expert in the field an do not limit the scope of the invention.

If plasma is to be analyzed, a smaller matrix bead size may be chosen e.g. in the range of 20 to 200 µm or a more narrow range within that range. If whole blood is to be analyzed, the size of matrix beads may be chosen in the range 100-300 µm or a more narrow range within said range.

The analysis plate used in the method according to the present invention is chosen by the expert in the field and this does not limit the scope of the invention. There are several different types of assay plates available having wells with a porous filter in the bottom of each well, allowing liquid to pass through, but not matrix beads or particles. Plates are available having for example 96 wells per plate, and plates having more wells are available, for example plates with 386 wells per plate. The larger number of wells can for example allow more samples to be determined or more different analytes to be determined, by using different matrix beads with different coupled ligands, than the plates with fewer wells. This can be determined by the expert in the field. Each well of said analysis plate has been provided with a porous matrix having one or more specific ligands bound thereto above the filter.

Preferably the matrix, to which one or more ligands are bound, has the form of porous gel particles. Further, the ligands are bound to the porous matrix both on the surface of the matrix and inside the pores of the matrix, thereby providing a larger surface available for binding. Alternatively, the matrix may have the form of a porous layer covering the whole or a part of the cross-section of each well and is in such a case located above the filter.

The pores in the porous matrix should preferably have such a porosity that also larger proteins, such as IgG, IgM (Immunoglobulin M) and toxins are allowed to enter into the pores and bind to the ligand, as well as enzyme-linked antibodies or enzyme-linked antigens should be able to enter the pores and bind to the analyte bound to the ligand within the pores.

The exact parameters for the matrix are chosen by the skilled person in the art, e.g. in view of the size of the beads and the volume of the beads applied in the wells. Typically a value of from 5 to 50 µl of beads with covalently bound ligand can be used per well. The expert in the field can determine the volume of matrix-ligand to be applied for the specific analysis to be performed.

Normally, when a beaded (e.g. in the form of gel particles) matrix is used, the matrix gel particle diameter lies in the interval of 20-300 µm, preferably 30-150 µm, but smaller smaller or larger particles can be chosen and also a more narrow interval of particle size distribution. Smaller particles will give a higher flow resistance than larger beads at the same bead volume. The pore size of the matrix, the volume of the bead layer, as well as the thickness of the gel bead layer, which shall allow a desired flow of liquid and/or sample through the filter in the bottom of the wells, can also be determined by a person skilled in the art.

The skilled person in the art can also determine the sample volume, the sample dilution for each well, the choice of ligand, choice of plate with filter and number and volume of the wells in the plate, the type of wash buffer, for example PA buffer, the amount of wash buffer, the type of enzyme-antibody or the type of enzyme-linked antigen, substrate, contact time for each step, flow rate through the filter and whether vacuum or centrifugation should be applied to speed up the flow of sample, wash buffer and other solutions through the beads and the filter for each step, as well as the time for the enzyme reaction to convert substrate to product.

Further, the skilled person in the art can also choose to apply a second mechanical filter applied above the matrix gel bead layer in each well with a view to reducing the problem with undesired agitation of the gel layer during the addition of the sample, the wash buffer, and the labelled antibody, e.g. enzyme-linked antibody, and substrate.

The skilled person in the art can also choose the amount of the gel beads of matrix-ligand, the amount of ligand to bind per volume of matrix, any dilution of the matrix-ligand with (not derivatized) matrix, the type and concentration of antibody label, the type of enzyme, the wash buffer, the type and concentration of substrate, the concentrations of other applied different reagents which according to the person skilled in the art can be used with the product and the method according to the invention.

In one embodiment of the detection method according to the present invention, the ligand is a saccharide and the matrix having a saccharide ligand bound thereto is used in an assay performed in an ELISA (Enzyme-Linked Immunosorbent Assay) plate. In the analysis device used in this embodiment the bottom in each well of the plate has been replaced with a mechanical filter having a predetermined pore diameter which allows passage of fluid but not of the matrix.

Below the filter in the bottom of each well of the plate, the analysis plate is in one embodiment designed as a downwards narrowing funnel starting from just below the filter of the well, with a view to leading/directing the liquid flow out through the filter below each well of the plate allowing the liquid to pass to the respective wells of a second plate placed below the analysis plate.

In this embodiment the ligand may also be a peptide or a protein covalently bound to the matrix.

In one embodiment the detection of the analyte is made with the basis of the reaction between an enzyme-linked antibody and a substrate, wherein the absorption of the reaction product is measured, either outside (the second plate mentioned above) or within the wells of the analysis plate. In one embodiment the ligand and the the antibody (or the antigen) labelled with an enzyme may be identical. In one embodiment, eluated reaction product is collected and measured in a container, e.g. an ELISA plate, or column arranged under the analysis plate.

To conclude, the method according to the present invention allows a substantially larger surface for the binding of the ligand and analyte compared to conventional ELISA. Also the method also allows for a higher volume of sample to be applied to each well than in ordinary ELISA, since the bead layer in each well act as a small affinity column allowing passage of one or several well volumes of sample through each well with matrix-ligand. This is important especially when determining low concentration of analyte in a sample. The sample is better contacted with the ligand compared to conventional ELISA since the analyte passes through narrow pores (for example with an average pore diameter in the size range of approximately 0.1 µm) with covalently bound ligand, thereby promoting binding between analyte and ligand. This in turn speeds up the binding of analyte compared to conventional ELISA. The sample volume applied is determined by the expert in the field. It also contributes to a reduction of the problem with non-specific binding.

In another embodiment of the invention the analysis plate with porous beads with covalently bound ligand as described and exemplified above, when the ligand is a saccharide, and is used in a kit for separation of analytes where the analyte is a saccharide binding biomolecule, for example a protein, an antibody, an other protein, such as for example a lectin or toxins, from a sample.

In this embodiment of the invention, the bound analyte can be eluted, after application of sample, binding and washing as described above, from the wells by for example addition of a buffer of higher pH (for example a pH of between 9 and 11) or lower pH (for example of a pH between 3 and 5), allowing the eluate to pass through the beads and the filter as described above, and collecting the eluted analyte, as described above. Alternatively, the analyte can be eluted by addition of a specific saccharide binding to the analyte, thus specifically eluting the analyte. In this way, for example a multiple purification of different analytes can be achieved, or the analyte can be purified from a range of samples. The analyte can then be used for different applications and/or for different types of analyses.

A method for the diagnosis of peptides and proteins, for example of antibodies specific for carbohydrate structures, is also disclosed. Several such antibodies are known specific for e.g. blood group antigens, for example blood group A, B, H, their different subtypes (for example blood group tetrasaccharides belonging to subtype 1, 2, 3 and 4), other blood group saccharides (such as blood group P and Pk), galili-antigens, TF-antigens and related antigens (for example Galβ1-3GalNAc structures), antibodies specific for other antigens of importance for example in autoimmune disorders, such as ganglioside carbohydrate structures (for example carbohydrate structures of GM1), for diagnosis of virus, bacteria, and toxins which have the ability to bind to carbohydrates as exemplified above, or to peptide receptors, antibodies specific for protein or peptide antigens, for example detection of antibodies specific for different HLA antigens. The method can also be used for example in determining the relative quantities of immunoglobulins IgG1, IgG2 and IgG3, IgA, and IgM which are specific for a certain antigen, for example blood group A and its subtypes, by using enzyme-labelled antibodies which are specific for the different types of human antibodies.

According to one embodiment of the present invention the saccharide is bound to the matrix via an aglycon. Non-limiting examples of the aglycon may e.g. be a monomeric aglycon glycosidically bound to the saccharide via -O-on the C1 position of the saccharide (i.e. the reducing end of the saccharide): -OPhNH-, OEtPhNH-, and O(CH)2-NH- are non-limiting examples of aglycons which may be chosen by the expert in the field, wherein the NH group is bound to the matrix, or bound to the matrix via a mono-, di-, oligo-, or polymer structure, e.g. a -(CH)n-O-CH2-matrix, wherein n is an integer, preferably 1,2,3,4,5, or 6.

Further non-limiting examples of the above-mentioned carbohydrates and carbohydrate derivatives according to the present invention, are one or several of blood group A-, blood group B-, blood group H- and or GM1, α-sialo-GM1, containing carbohydrates found in glycoproteins, glycopeptides, or glycolipids, mono-, di-, tri-, tetravalent and higher oligomers of above mentioned A-, B-, H- and or GM1 and or α-sialo-GM1-oligosaccharides, derivatives of said saccharides e.g. containing O-, N-, or S-glycosides of these substances, wherein the aglycon comprises e.g. an aliphatic or aromatic part and e.g. the aglycon containing a terminal sulfhydryl-, hydroxyl-, amino- or carboxyl group for covalent binding to the matrix, e.g. the polymer particle, bead or filter as described above, or in which said carbohydrate derivatives contains at least one biotin, avidin or streptavidin molecule for non-covalent binding to an avidine, streptavidine or biotin-matrix.

The choice of the aglycon and its binding to saccharide or peptide and its binding to the matrix is determined by the expert in field and does not limit the scope of the invention.

A di-, tri-, tetra-, or oligomeric aglycon can be constructed by the expert in the field to be able to be linked O-, N- or S-glycosidically, or via another aglycon in between the carbohydrate and the -(CH₂)ₙ-O- units, to one or serveral carbohydrates or carbohydrate derivatives, thereby forming a dimeric, tri-meric, tetrameric, or oligomeric carbohydrate derivative, which in turn is linked to matrix.

The di-, tri-, tetra- or oligomeric ligand can be chosen by the expert to obtain a stronger binding to the product of the antibody or protein to be analysed and/or minimize the size of the product for the specific application/use of the product. The exact structure of the aglycon is made by the expert in the field. The aglycon can also contain a peptide or protein.

The quantity of ligand in the matrix-ligand is typically chosen to contain 0.1, 0.5, 1.0, 2.0, 10, or 20 mg ligand per mL volume of matrix-ligand or is any value between these values. The exact value is chosen by the expert in the field.

At least the final stages of the production of the matrix-ligand and also the application of matrix-ligand in the wells can optionally be performed in clean rooms, and the reagents and clean room(s) used are preferentially certified according to international standards and/or requirements for the product application.

Alternatively, the matrix-ligand is end-sterilized with steam and or autoclaving to ensure a sterile matrix-ligand product before use.

The pores in the matrix should preferably have such a porosity that also larger proteins, such as IgM (Immunoglobulin M) and toxins are allowed to enter into the pores, as well as enzyme-linked antibodies.

The enzyme and the conjugate enzyme-antibody or enzyme-antigen, as well as the concentration and quantity of the conjugate per weill, are chosen by the expert in the field and do not limit the scope of the invention. For example, if a human antibody is to be determined by the method according to the invention, a conjugate between the enzyme and the anti-human antibody is used, wherein the anti-human antibody is produced in for example a rabbit or mouse. The enzyme-antibody conjugate can, also according to the present invention, be a conjugate between an enzyme and a fragment of an antibody which is able to bind the analyte.

The enzyme can for example be a peroxidase or an alkaline phosphatase. The substrate, its concentration, the buffer solution with substrate and its volume per well, as well as the choice of enzyme is made by the expert in the field and does not limit the scope of the invention.

For example, when determining the quantity of blood group specific antibodies in a plasma sample, the following components can be chosen:
A known volume of matrix-ligand, e.g. a beaded agarose with covalently bound blood group A saccharide, for binding of the blood group A specific antibody in the plasma, is added to each well. Plasma samples, each one of the same volume but of different dilutions, are added to the wells, and binding is allowed to take place. Non-bound plasma components are washed with wash buffer and are allowed to pass through the beads and the filter in the bottom of each well. The wash procedure is repeated, typically one to three times, with a view to removing practically all non-bound components. A diluted conjugate of peroxidase-labelled anti-human IgG is added to each well. After binding of the conjugate for a predetermined time, a wash solution is added to each well, and non-bond conjugates follow the wash solution through the filter in each well. This is repeated, typicallly one to three times, to remove practically all non-bound conjugates. The same volume and concentration of peroxidase substrate solution, OPD, is added to each well. The enzyme raction is allowed to take place. A second plate with corresponding wells is placed under the respective wells of the above-mentioned assay plate. Centrifugation or vacuum are applied to allow the product solution to pass through the gel and the filter of the assay plate down to the corresponding wells of the second plate. An ELISA reader is used to read the absorption (e.g. at 492 nm) of each well.

Curves (absorption against dilution) for the dilution series of each type of sample of plasma applied is obtained and the quantity of antibody is determined (for example using results for a known quantity of purified human antibody specific for the same ligand).

A good correlation with the anti-A titers (obtained by titration against red blood cells) in different pooled or non-pooled plasma samples of higher or lower titers from different blood donors was obtained using the product and method above. Samples with higher titers gave higher absorption values (more product formation) than samples of lower titers. As matrix-ligand, i.e. blood group A-Sepharose was used, that is cross-linked agarose with covalently bound blood group A determinant. Samples (used as a control) which had passed the A-Sepharose gave low or no binding.

One of the advantages with the method according to the present invention is for example that a more absolute measure of the specific antibody is obtained than with conventional red blood cell titrations. Moreover, plasma samples from several blood donors or from several patients can be screened on the same plate. Also, by using matrix with covalently bound A-trisaccharide and matrixes with covalently bound A-tetrasaccharide of the different blood group A subtypes, more information about the quantity and specificity of the blood group specific antibodies in the sample can be obtained than by using conventional red blood cell titration.

Moreover, by using peroxidase conjugated to anti-human IgG1, peroxidase conjugated to anti-human IgG2, and peroxidase conjugated to anti-human IgG3, the content of for example IgG1, IgG2 and IgG3 specific for the blood group A saccharides mentioned above, or specific for other saccharides, peptides or proteins (used as ligands in the assay), can be determined for a range of blood group subtypes, as well as for other ligands, in one or a few assay plates.

The concentrations, dilutions and volumes of reagents above, as well as contact times for binding, washing and enzyme reactions, and other parameters, are determined by the person skilled in the art.

Typically, 10 µl or 50 µl or a value between these values, of matrix-ligand in each well and a few minutes of binding (typically between 2 and 10 minutes or a value between these values, for example 5 minutes) and washing has been shown to be sufficient for each one of the steps above, except for the binding of the enzyme-antibody conjugate, or for the enzyme-antigen conjugate, and for the enzyme reaction, which normally requires a longer time, typically 10 minutes or 15 minutes, or a value between these values. All steps can optionally be performed at room temperature.

As a further example may be mentioned that by using matrix with different bound ligands, in each well or in different lines of wells, multiple analytes can be determined, for example for screening of antibodies of diffferent specificities towards, for example, but not limited to, different carbohydrate antigens, towards different HLA antigens, or towards different toxins.

### Example

In the following a non-limiting example of an embodiment of the present invention is disclosed. After the application of a sample containing an analyte to be determined to at least one well of an ELISA plate containing matrix-ligand, non-bound material is washed away by the addition of a wash buffer, e.g. a PA buffer, which is allowed to pour down through the porous matrix and the filter. Thereafter, a solution containing enzyme-linked antibody is added, wherein said antibody has the ability to bind to the analyte. This is followed by a washing step with a wash buffer with a view to washing away non-bound enzyme-labelled antibody, and a substrate is then added to each well. The product formed in each well is measured spectrophotometrically by an ELISA plate reader, either directly or after suction of the product solution in each well to the corresponding wells of an ELISA plate.

## Claims

1. A method for the detection of at least one analyte in a sample, wherein it comprises the steps of:
a) providing a layer comprising a porous matrix having the form of several porous agarose gel beads and having ligands bound to the porous matrix both on the surface of the matrix and inside the pores of the matrix above a filter constituting the bottom of several wells of an ELISA plate, wherein the pores of said porous agarose gel beads allow entrance of proteins or antibodies up to at least one million Daltons into said pores, wherein said filter does not allow passage of said porous matrix having a ligand bound thereto and wherein a part of the ELISA plate below the filter has a funnel shape narrowing downwards to a smaller opening,
b) adding a sample containing said at least one analyte to be detected, said at least one analyte having the ability to specifically bind to said at least one ligand,
c) adding a wash solution with a view to washing out non-bound sample components from the ELISA plate through the filter,
d) adding enzyme-linked antibodies or antigens having the ability to specifically bind to said at least one analyte,
e) adding a wash solution with a view to washing out non-bound enzyme-linked antibodies or antigens from the analysis plate through the filter,
f) adding a substrate specific for the enzyme, wherein a product is formed by a reaction between the enzyme and the substrate, wherein the product formed is either subjected to direct spectrophotometic measurement of the adsorption of the wells in the ELISA plate or is washed from the well with a wash solution through the filter in the bottom of the well and is collected as an eluate below the ELISA plate,
g) determining the quantity of said at least one analyte by measuring a signal related to the product.

2. The method according to claim 1, wherein each ligand is covalently bound to the matrix.

3. The method according to claim 1 or 2, wherein different ligands are bound to each matrix with a view to detecting different analytes in the sample or in more than one sample.

4. The method according to any one of the preceding claims, wherein the agarose is cross-linked agarose, more preferably CL-Sepharose 2B, 4B or 6B.

5. The method according to claim 2 wherein the ligand comprises an antigen, preferably a saccharide, optionally bound to the matrix via an aglycon, a protein or a peptide, or an antibody having the ability to specifically bind to the analyte in the sample.

6. The method according to claim 5, wherein the saccharide contains at least one of D-galactose, N-acetyl-D-galactosamine, N-acetyl-D-glucosamine, L-fucose, D-glucose, and sialic acid.

7. The method according to claim 5, where the saccharide is Galα1-4Gal or Galα1-4Galβ1-4Glc.

8. The method according to any one of the preceding claims, wherein said at least one analyte is a Shiga toxin.

9. The method according to any one of the preceding claims, wherein a further filter is applied above the layer of said porous matrix.

10. The method according to claim 1, wherein the size of the agarose gel beads is 20-300 µm, preferably 30-200 µm.

11. A method for the diagnosis of an EHEC (enterohemorrhagic Escherichia coli) infection or HUS (hemolytic uremic syndrome), wherein a body sample from a patient is added as the sample and the analyte is detected using the method according to any one of claims 1-10, wherein the ligand contains the saccharide Galα1-4Gal or Galα1-4Galβ1-4Glc.

## Patentansprüche

1. Verfahren zum Nachweis mindestens eines Analyten in einer Probe, wobei es die folgenden Schritte umfasst:
a) Bereitstellen einer Schicht, umfassend eine poröse Matrix, die die Form mehrerer poröser Agarosegelkügelchen hat und wobei an die poröse Matrix Liganden sowohl an der Oberfläche der Matrix als auch im Inneren der Poren der Matrix gebunden sind, oberhalb eines Filters, das den Boden mehrerer Vertiefungen einer ELISA-Platte bildet, wobei die Poren der porösen Agarosegelkügelchen den Eintritt von Proteinen oder Antikörpern bis zu mindestens einer Million Dalton in die Poren gestatten, wobei das Filter nicht den Durchtritt der porösen Matrix mit einem daran gebundenen Liganden gestattet und wobei ein Teil der ELISA-Platte unterhalb des Filters eine Trichterform hat, die sich nach unten zu einer kleineren Öffnung verjüngt,
b) Hinzufügen einer Probe, die den mindestens einen nachzuweisenden Analyten enthält, wobei der mindestens eine Analyt die Fähigkeit besitzt, spezifisch an den mindestens einen Liganden zu binden,
c) Hinzufügen einer Waschlösung im Hinblick auf das Auswaschen nicht-gebundener Probenbestandteile aus der ELISA-Platte durch das Filter,
d) Hinzufügen Enzym-gebundener Antikörper oder Antigene, die die Fähigkeit besitzen, spezifisch an den mindestens einen Analyten zu binden,
e) Hinzufügen einer Waschlösung im Hinblick auf das Auswaschen nicht-gebundener Enzym-gebundener Antikörper oder Antigene aus der Analyseplatte durch das Filter,
f) Hinzufügen eines für das Enzym spezifischen Substrats, wobei ein Produkt durch eine Reaktion zwischen dem Enzym und dem Substrat gebildet wird, wobei das gebildete Produkt entweder einer direkten spektralphotometrischen Messung der Adsorption der Vertiefungen in der ELISA-Platte unterzogen oder aus der Vertiefung mit einer Waschlösung durch das Filter im Boden der Vertiefung ausgewaschen und als Eluat unterhalb der ELISA-Platte gesammelt wird,
g) Bestimmen der Menge des mindestens einen Analyten durch Messen eines Signals, das zu dem Produkt in Beziehung steht.

2. Verfahren nach Anspruch 1, wobei jeder Ligand kovalent an die Matrix gebunden ist.

3. Verfahren nach einem der Ansprüche 1 oder 2, wobei verschiedene Liganden an jede Matrix im Hinblick auf das Nachweisen verschiedener Analyten in der Probe oder in mehr als einer Probe gebunden sind.

4. Verfahren nach einem der vorhergehenden Ansprüche, wobei die Agarose vernetzte Agarose, stärker bevorzugt CL-Sepharose 2B, 4B oder 6B, ist.

5. Verfahren nach Anspruch 2, wobei der Ligand ein Antigen, vorzugsweise ein Saccharid, das gegebenenfalls über ein Aglykon, ein Protein oder ein Peptid an die Matrix gebunden ist, oder einen Antikörper mit der Fähigkeit, spezifisch an den Analyten in der Probe zu binden, umfasst.

6. Verfahren nach Anspruch 5, wobei das Saccharid mindestens eines aus D-Galactose, N-Acetyl-D-galactosamin, N-Acetyl-D-glucosamin, L-Fucose, D-Glucose und Sialinsäure enthält.

7. Verfahren nach Anspruch 5, wobei das Saccharid Galα1-4Gal oder Galα1-4Galβ1-4Glc ist.

8. Verfahren nach einem der vorhergehenden Ansprüche, wobei der mindestens eine Analyt ein Shiga-Toxin ist.

9. Verfahren nach einem der vorhergehenden Ansprüche, wobei ein weiteres Filter oberhalb der Schicht der porösen Matrix aufgebracht wird.

10. Verfahren nach Anspruch 1, wobei die Größe der Agarosegelkügelchen 20-300 µm, vorzugsweise 30-200 µm, beträgt.

11. Verfahren zur Diagnose einer EHEC(enterohämorrhagische Escherichia coli)-Infektion oder von HUS (hämolytisch-urämischem Syndrom), wobei eine Körperprobe von einem Patienten als die Probe zugegeben wird und der Analyt unter Verwendung des Verfahrens nach einem der Ansprüche 1-10 nachgewiesen wird, wobei der Ligand das Saccharid Galα1-4Gal oder Galα1-4Galβ1-4Glc enthält.

## Revendications

1. Méthode de détection d'au moins un analyte dans un échantillon, où ladite méthode comprend les étapes consistant à :
a) se munir d'une couche comprenant une matrice poreuse ayant la forme de plusieurs billes poreuses de gel d'agarose et ayant des ligands liés à la matrice poreuse, à la fois à la surface de la matrice et à l'intérieur des pores de la matrice au-dessus d'un filtre constituant le fond de plusieurs puits d'une plaque ELISA, où les pores desdites billes poreuses de gel d'agarose permettent l'entrée des protéines ou des anticorps jusqu'à au moins un million de daltons dans lesdits pores, où ledit filtre ne permet pas le passage de ladite matrice poreuse à laquelle un ligand est lié et où une partie de la plaque ELISA en dessous du filtre présente une forme d'entonnoir qui s'étrécit vers le bas jusqu'à une ouverture plus petite,
b) ajouter un échantillon contenant ledit au moins un analyte à détecter, ledit au moins un analyte ayant la capacité de se lier spécifiquement audit au moins un ligand,
c) ajouter une solution de lavage dans le but d'entraîner les composants d'échantillon non liés de la plaque ELISA à travers le filtre,
d) ajouter des anticorps liés à des enzymes ou des antigènes ayant la capacité de se lier spécifiquement audit au moins un analyte,
e) ajouter une solution de lavage dans le but d'entraîner les anticorps liés à des enzymes ou les antigènes non liés de la plaque d'analyse à travers le filtre,
f) ajouter un substrat spécifique de l'enzyme, où un produit est formé par une réaction entre l'enzyme et le substrat, où le produit formé est soumis à une mesure spectrophotométrique directe de l'adsorption des puits dans la plaque ELISA ou est entraîné hors du puits avec une solution de lavage à travers le filtre situé au fond du puits avant d'être recueilli sous forme d'éluat en dessous de la plaque ELISA,
g) déterminer la quantité dudit au moins un analyte par mesure d'un signal lié au produit.

2. Méthode selon la revendication 1, où chaque ligand est lié de façon covalente à la matrice.

3. Méthode selon la revendication 1 ou 2, où différents ligands sont liés à chaque matrice dans le but de détecter différents analytes dans l'échantillon ou dans plusieurs échantillons.

4. Méthode selon l'une quelconque des revendications précédentes, où l'agarose est de l'agarose réticulé, plus préférentiellement CL-Sepharose 2B, 4B ou 6B.

5. Méthode selon la revendication 2, où le ligand comprend un antigène, préférentiellement un saccharide, éventuellement lié à la matrice via un aglycone, une protéine ou un peptide, ou un anticorps ayant la capacité de se lier spécifiquement à l'analyte dans l'échantillon.

6. Méthode selon la revendication 5, où le saccharide contient au moins l'un des membres du groupe constitué par les suivants : D-galactose, N-acétyl-D-galactosamine, N-acétyl-D-glucosamine, L-fucose, D-glucose et acide sialique.

7. Méthode selon la revendication 5, où le saccharide est Galα1-4Gal ou Galα1-4Galβ1-4Glc.

8. Méthode selon l'une quelconque des revendications précédentes, où ledit au moins un analyte est une shigatoxine.

9. Méthode selon l'une quelconque des revendications précédentes, où un filtre supplémentaire est appliqué au-dessus de la couche de ladite matrice poreuse.

10. Méthode selon la revendication 1, où la taille des billes de gel d'agarose est de 20 à 300 µm, préférentiellement de 30 à 200 µm.

11. Méthode de diagnostic d'une infection par EHEC (Escherichia coli entérohémorragique) ou du syndrome hémolytique et urémique (SHU), où un échantillon corporel issu d'un patient est ajouté au titre d'échantillon et l'analyte est détecté en utilisant la méthode selon l'une quelconque des revendications 1 à 10, où le ligand contient le saccharide Galα1-4Gal ou Galα1-4Galβ1-4Glc.
